# NOUVEAU FASCICULE DE BREVET EUROPEEN

(11) **EP 0 862 468 B2**
(45) Date de publication et mention de la décision concernant l'opposition: **23.07.2008**
(45) Mention de la délivrance du brevet: 06.05.2004
(21) Numéro de dépôt: 97944936.0
(22) Date de dépôt: 07.10.1997
(51) Int. Cl.: A61L 24/00, C09J 189/06, C09J 103/10

(54) **COMPOSITION ADHESIVE A BASE DE POLYALDEHYDE MACROMOLECULAIRE ET PROCEDE DE RETICULATION DE COLLAGENE**
KLEBSTOFFZUSAMMENSETZUNG AUF DER BASIS VON HOCHMOLEKULAREM POLYALDEHYD UND VERFAHREN ZUR VERNETZUNG VON KOLLAGEN
ADHESIVE COMPOSITION WITH MACROMOLECULAR POLYALDEHYDE BASE AND METHOD FOR CROSS-LINKING COLLAGEN

(30) Priorité: 07.10.1996 FR 9612200; 23.12.1996 FR 9615888
(43) Date de publication de la demande: 09.09.1998
(73) Titulaire: Imedex Biomateriaux, 69630 Chaponost (FR)
(72) Inventeur: TARDY, Michel, F-69005 Lyon (FR); VOLCKMANN, Hervé, F-69005 Lyon (FR); TIOLLIER, Jérôme, F-69005 Lyon (FR); GRAVAGNA, Philippe, F-69540 Irigny (FR); TAYOT, Jean-louis, F-69890 La Tour de Salvagny (FR)
(74) Mandataire: Bernasconi, Jean Raymond
(86) Numéro de dépôt international: PCT/FR1997/001787
(87) Numéro de publication internationale: WO 1998/015299

(56) Documents cités:
- US-A- 3 057 723
- US-A- 4 339 360
- US-A- 5 292 362
- US-A- 5 385 606
- E. SCHACHT ET AL.: "Some aspects of the crosslinking of gelatin by dextran dialdehydes" POLYMER GELS AND NETWORKS, vol. 1, 1993, GB, pages 213-224, XP000677632
- Y. NAYUDAMMA ET AL.: "Investigation on the interaction of dialdehydes and polyaldehydes on collagen" J. AMER. LEATHER CHEM. ASS., vol. 64, no. 9, 1969, USA, pages 444-452, XP002052831
- CHEMICAL ABSTRACTS, vol. 116, no. 10, 9 mars 1992 Columbus, Ohio, US; abstract no. 85407, "Gelatin-based adhesives for bookbinding" XP002034188 & JP 03 239 781 A (NITTA GELATIN INC.) 25 octobre 1991
- H. Onishi, T. Nagai, International Journal of Pharmaceutics 1986, 30, pp. 133-141

## Description

La présente invention se situe dans le domaine des colles biologiques biodégradables et non toxiques destinées à un usage chirurgical ou thérapeutique.

D'une manière plus précise, la présente invention a trait à une composition adhésive biocompatible, biorésorbable et non toxique, à base d'au moins un polyaldéhyde macromoléculaire, selon la revendication 1 ou 2.

Elle concerne également un procédé de réticulation de collagène solubilisé permettant d'obtenir un matériau adhésif destiné à être appliqué rapidement sur des tissus et/ou un biomatériau.

La réticulation du collagène peut être réalisée soit par voie chimique, à l'aide d'agents tannants tels que le glutaraldéhyde ou le formaldéhyde ou encore le diisocyanate ou d'autres réactifs, soit par des agents physiques tels que les radiations gamma, béta ou ultraviolettes.

Cependant, cette dernière méthode est lourde et parfois difficile à mettre en oeuvre et, outre les réticulations, elle provoque aussi des coupures.

En ce qui concerne la voie chimique, le traitement par le glutaraldéhyde (ou le formaldéhyde), est le traitement le plus souvent utilisé pour réticuler le collagène et consiste à immerger dans une solution de glutaraldéhyde des poudres, des films, des gels ou des solutions plus ou moins concentrées en collagène. Il présente un certain nombre d'inconvénients selon les applications. L'introduction de glutaraldéhyde dans une structure collagénique aqueuse peut notamment entraîner un relargage rapide de glutaraldéhyde en excès, par diffusion à travers le gel formé.

En chirurgie ou thérapie, ceci est à l'origine de réactions toxiques, entraînant des nécroses tissulaires, ou des réactions moins sévères, conduisant à une mauvaise cicatrisation ou à son ralentissement. C'est le cas de l'adhésif collagénique aujourd'hui disponible sur le marché.

Dans cette colle, nommée GRF, la réticulation de la gélatine est réalisée par le formaldéhyde en présence de résorcinol, ce dernier servant essentiellement à réduire la dissolution de la masse adhésive. Cette colle a été utilisée entre les années 60 et les années 80 mais en fait son emploi en chirurgie est maintenant restreint à quelques applications rares où le bénéfice l'emporte sur les risques (dissection aortique) en raison de la possibilité de relargage du formaldéhyde et de sa toxicité. Une quantité excessive de formaldéhyde est nécessaire pour obtenir une réticulation rapide du gel collagénique et une bonne adhésion aux tissus environnants. Cet excès de formaldéhyde est responsable de la mauvaise biocompatibilité de la colle GRF. On ne peut donc généraliser aujourd'hui l'emploi de cette colle à la cicatrisation des plaies chirurgicales ou chroniques, à la protection ou l'étanchéité de sutures ou encore à la délivrance de médicaments.

Une amélioration récente vient d'être proposée en augmentant la viscosité du milieu qui contient le formaldéhyde, le glutaraldéhyde ou autre dialdéhyde, par addition d'un gelifiant tel que l'agarose (Demande de brevet IZORET G., WO 96/14368). Cependant, ceci ne supprime pas le risque de diffusion de l'agent réticulant même si la diffusion est ralentie.

Par ailleurs, la biocompatibilité du collagène réticulé par des agents chimiques toxiques peut être améliorée en réduisant au minimum la quantité d'agents réticulants employés. C'est ce qui est réalisé dans des matériaux préformés destinés à être implantés, où le temps de réaction peut être allongé autant que nécessaire par le fabricant du matériau. Dans ce cas, de faibles quantités d'agent réticulant sont employées et le matériau réticulé est lavé soigneusement en fin de préparation pour éliminer tout excès de réactif.

Le matériau réticulé final utilisé par le chirurgien est dans ce cas un solide ou une suspension de fibres collagéniques, mais ces matériaux sont dépourvus d'adhésivité aux tissus environnants.

L'obtention d'une colle biologique nécessite souvent le mélange, par le chirurgien lui-même, au contact des tissus biologiques à traiter, de deux solutions différentes permettant une réticulation rapide du collagène, de la gélatine ou de tout autre macromolécule ou molécule réactive.

On connait par exemple la colle commercialisée par la Société IMMUNO sous le nom de "TISSUCOL" (TISSEEL) puis par BEHRINGWERKE sous le nom de "BERIPLAST" et par BIOTRANSFUSION sous le nom de "BIOCOL". Il s'agit d'une solution concentrée de fibrinogène (70-140 mg/ml) contenant du facteur XIII et de la fibronectine dont la polymérisation est induite par une solution de thrombine (4 à 400 Unités Internationales) dans un mélange extemporané. Le fibrinogène polymérise ensuite en fibrine pour reformer un coagulum qui assure l'adhésion des tissus mis en contact.

Les difficultés et problèmes majeurs soulevés par ce produit et ses composants sont d'une part, l'absence de caractérisation complète et de reproductibilité de la quantité de chacun des composants de la solution de fibrinogène (Facteur XIII, fibronectine, aprotinine) ; et d'autre part, la difficulté d'inactivation virale d'un tel produit vis-à-vis des virus non enveloppés comme des Agents Transmissibles Non Conventionnels. Ceci a conduit à une limitation de la possibilité d'utiliser de tels produits à grande échelle.

TARDY et Coll., (Demande de brevet français n° 9400715), ont décrit un adhésif biologique collagénique qui peut être préparé grâce à un kit constitué par exemple de deux seringues séparées contenant respectivement une solution de collagène (ou gélatine) oxydé par le périodate de sodium et conservé à pH acide sous forme congelée à une température inférieure à 0°C, de préférence de l'ordre de -20°C, et une solution alcaline aqueuse. Le mélange des contenus respectifs est assuré par un mélangeur connecté aux deux seringues, après réchauffage du gel de collagène (ou gélatine) oxydé aux environs de 40°C, pour obtenir un adhésif biocompatible dont la réticulation est accomplie en 2 à 3 minutes.

Les propriétés de cet adhésif sont intéressantes dans certaines applications, mais le problème principal de cette technologie est la nécessité d'une chaîne de froid complexe pour la distribution de ce produit, ce qui en augmente le coût et en gêne l'emploi dans des locaux non équipés de congélateur.

On a également proposé d'utiliser des dérivés réactifs du polyéthylène glycol pour former des colles biologiques à base d'albumine ou de collagène (Brevet BARROWS et Col. - n° WO 96 031-59 A1 ; SIERRA D. - Tissue Sealants Meeting La Jolla, 1996). Cependant, ces réactifs sont peu stables dans l'eau et peuvent nécessiter des modes de conservation particuliers. Leur pH optimum d'activité est alcalin, non physiologique. D'autre part, le temps de résorption de ces produits est très long, supérieur à 3 à 4 semaines, ce qui, dans certaines applications, est un inconvénient majeur.

La nécessité incontournable d'obtenir rapidement et quelquefois presque instantanément, la solidification d'une solution fluide pour obtenir une adhésion forte, peut rendre nécessaire l'emploi d'un excès de réactifs chimiques de réticulation qui provoque la toxicité et la médiocre biocompatibilité tissulaire.

Des colles tissulaires à base de gélatine commerciale et de glutaraldéhyde ou d'amidon oxydé ont aussi été récemment proposées dans le document WO 97/29715 (Fusion Médical Technologies, Inc.).

Ces colles forment des gels très visqueux qui doivent être chauffés à température élevée, de l'ordre de 50-80°C, pour leur application à la seringue.

Outre le risque de toxicité potentiel selon l'aldéhyde utilisé, ces colles peuvent endommager les tissus traités notamment du fait de leur température d'application.

L'invention a pour objectif de fournir une composition adhésive ne présentant pas les inconvénients majeurs évoqués précédemment.

Elle a ainsi pour objectif de fournir une composition adhésive biocompatible, biorésorbable et non toxique, adaptée à un usage chirurgical et/ou thérapeutique, qui soit stable dans le temps et pouvant être conservée dans des conditions relativement simples tout en étant d'utilisation aisée, notamment injectable à l'aide d'aiguilles ou de cathéters.

Elle a encore pour objectif de fournir une composition adhésive présentant des propriétés d'adhésion et de résistance mécanique améliorées.

Un autre objectif de l'invention est en particulier de fournir une composition adhésive avec laquelle la réticulation du collagène ou de la gélatine s'effectue très rapidement et dont la vitesse puisse être facilement modulée.

Un autre objectif est encore de fournir une composition adhésive ne présentant pas de risque de toxicité en particulier par diffusion de l'agent réticulant.

Un objectif de l'invention est aussi de fournir une composition adhésive pour la liaison de tissus biologiques, y compris des tissus vivants, entre eux ou avec un biomatériau implanté qui peut être l'adhésif lui-même (utilisé seul).

L'invention a par ailleurs pour objectif de fournir un procédé de réticulation du collagène (ou gélatine) permettant l'obtention notamment d'une telle composition adhésive, qui soit facile à mettre en oeuvre et sans danger pour l'organisme receveur.

L'invention a encore pour objectif de fournir des kits comprenant une composition adhésive telle que précitée, à usage chirurgical et/ou thérapeutique, d'utilisation simple et pratique.

A cette fin, l'invention a pour objet une composition adhésive biocompatible, biorésorbable et non toxique, à usage chirurgical et/ou thérapeutique, notamment pour la liaison de tissus biologiques entre eux ou à un biomatériau implanté, comprenant au moins un polysaccharide ou un mucopolysaccharide oxydé polyaldéhydique macromoléculaire, en solution aqueuse au pH acide naturellement obtenu.

On pense que la stabilité de telles solutions aqueuses est obtenue, de façon naturelle, par le fait que la solution devient spontanément acide.

L'invention a aussi pour objet une composition adhésive, caractérisée en ce qu'elle comprend : d'une part, un composant collagénique solubilisé en milieu aqueux, choisi parmi
- le collagène ayant perdu au moins partiellement sa structure hélicoïdale, non hydrolysé, constitué majoritairement de chaînes α,
- le collagène natif selon une concentration inférieure à 5 %, et
d'autre part, au moins un polysaccharide ou mucopolysaccharide oxydé polyaldéhydique macromoléculaire biodégradable d'origine naturelle.

L'invention fournit également un procédé de préparation d'une colle biocompatible, biorésorbable et non toxique, à usage chirurgical et/ou thérapeutique, destinée à être appliquée sur des tissus et/ou un biomatériau, caractérisé en ce qu'il comprend l'étape consistant à mélanger à un composant collagénique solubilisé en milieu aqueux, choisi parmi
- le collagène ayant perdu au moins partiellement sa structure hélicoïdale, non hydrolysé, constitué essentiellement de chaînes α,
- le collagène natif selon une concentration inférieure à 5 %,
préalablement à sa réticulation, au moins un polysaccharide ou mucopolysaccharide oxydé polyaldéhydique macromoléculaire biodégradable d'origine naturelle en solution aqueuse.

L'invention a encore pour objet l'utilisation d'un polysaccharide ou mucopolysaccharide oxydé polyaldéhydique macromoléculaire biodégradable d'origine naturelle en solution aqueuse pour l'obtention d'une composition adhésive biocompatible, biorésorbable et non toxique, à usage chirugical et/ou thérapeutique pour la liaison de tissus biologiques entre eux ou à un biomatériau implanté présentant des fonctions réactives aminées vis-à-vis dudit polyaldéhyde.

L'invention a encore pour objet l'utilisation d'un tel polyaldéhyde en combinaison avec un composant collagénique tel que précédemment défini, en solution aqueuse pour l'utilisation ci-dessus.

Enfin l'invention a pour objet des kits pour composition adhésive biocompatible, biorésorbable et non toxique, destinés à un usage chirurgical et/ou thérapeutique, caractérisés en ce qu'ils comprennent une composition selon la revendication 1.

L'invention a aussi pour objet des kits comprenant en outre :
- une solution aqueuse d'un composant collagénique choisi parmi
   . le collagène ayant perdu au moins partiellement sa structure hélicoïdale, non hydrolysé, constitué majoritairement de chaînes α,
   . le collagène natif selon une concentration inférieure à 5 %;
- des moyens de mélange pour mélanger extemporanément lesdites solutions.

L'utilisation selon la revendication 20 convient pour l'application à une température comprise entre 20 et 45°C sur lesdits tissus et/ou sur ledit biomatériau, ledit biomatériau présentant des fonctions, notamment aminées, réactives vis-à-vis dudit polyaldéhyde.

L'utilisation selon la revendication 21 convient pour une application telle que :
- on mélange à pH neutre, physiologique, une solution aqueuse d'un composant collagénique choisi parmi:
   . le collagène ayant perdu au moins partiellement sa structure hélicoïdale, non hydrolysé, constitué majoritairement de chaînes α;
   . le collagène natif selon une concentration inférieure à 5 %;
   et une solution aqueuse contenant au moins un polyaldéhyde macromoléculaire biodégradable d'origine naturelle, tel que défini précédemment;
- on applique rapidement le gel obtenu sur lesdits tissus et/ou ledit biomatériau, celui-ci présentant des fonctions, notamment aminées, réactives vis-à-vis desdits polyaldéhydes macromoléculaires, à une température comprise entre 20 et 45°C; et
- on laisse polymériser ledit mélange.

Les inventeurs ont découvert, de manière tout à fait inattendue, les propriétés potentiellement adhésives de tels polyaldéhydes macromoléculaires biodégradables d'origine naturelle sur les tissus vivants et l'aptitude des adhésifs qui en découlent, à être extrêmement bien tolérés par l'organisme receveur tout en conservant leurs propriétés adhésives pendant leur séjour dans l'organisme.

Ils ont ainsi découvert que l'on pouvait coller des tissus biologiques, y compris des tissus vivants, entre eux ou avec un biomatériau implanté, celui-ci présentant des fonctions réactives vis-à-vis des polyaldéhydes macromoléculaires, notamment des fonctions aminées.

Les inventeurs ont découvert de manière surprenante que l'on pouvait obtenir une colle biologique en apportant l'excès de réactifs chimiques nécessaire à la réticulation de certains collagènes ou gélatines sous forme d'un tel polyaldéhyde macromoléculaire biodégradable et que cette colle est efficacement applicable sur les tissus vivants à une température proche de la température physiologique, n'entraînant pas de lésion tissulaire, et présente des propriétés améliorées notamment au niveau de la qualité d'adhésion et de la résistance mécanique.

Selon l'invention, par "polyaldéhyde macromoléculaire biodégradable d'origine naturelle", on entend tout composé présentant plusieurs fonctions aldéhydiques, dérivé d'un polymère naturel biodégradable.

Le terme "biodégradable" désigne un polyaldéhyde macromoléculaire susceptible de disparaître par dégradation progressive (métabolisation).

Le polyaldéhyde macromoléculaire peut être aisément préparé par oxydation de polysaccharides ou de mucopolysaccharides avec notamment l'acide périodique ou ses sels, selon un procédé connu en soi depuis très longtemps. De telles préparations de polysaccharide oxydé ont déjà été autrefois proposées pour réticuler et stabiliser des matériaux collagéniques solides dans des documents anciens (brevet US-A-3 093 439 Johnson & Johnson et GB-A-1 109 509 Unilever), ou de la gélatine solubilisée en milieu tamponné

Or, les inventeurs ont découvert qu'en utilisant un tel agent réticulant, on n'observait pas le phénomène de diffusion de celui-ci, phénomène très problématique dans les colles biologiques connues, notamment la colle GRF, en particulier du point de vue de la toxicité et de la biocompatibilité tissulaire.

Ils ont découvert en particulier que le polyaldéhyde macromoléculaire, en apportant la quantité nécessaire d'aldéhyde dans la solution de collagène ou de gélatine, va se trouver emprisonné à l'intérieur du gel adhésif qu'il aura permis de former.

Le polyaldéhyde macromoléculaire mis en oeuvre selon l'invention consiste avantageusement en un polysaccharide ou mucopolysaccharide oxydé.

Le polysaccharide possède avantageusement un poids moléculaire compris entre 10 000 et 2 millions Daltons.

Parmi les polysaccharides ou mucopolysaccharides convenant à la réalisation de l'invention, on peut citer l'amidon, le dextrane, l'agarose, la cellulose, la chitine, le chitosan, l'acide alginique, les glycosaminoglycanes, l'acide hyaluronique et le chondroïtine sulfate ou leurs dérivés.

Selon l'invention, l'amidon et le dextrane sont préférés, l'amidon étant tout particulièrement préféré.

Les polyaldéhydes peuvent être utilisés seuls ou en mélanges.

Le terme "polyaldéhyde" utilisé selon l'invention désigne indifféremment un polyaldéhyde seul ou un mélange de plusieurs polyaldéhydes.

Conformément à l'invention, le polyaldéhyde peut résulter de l'oxydation d'un composé précité par l'acide périodique ou l'un de ses sels, de préférence le périodate de sodium, selon des procédé connus en soi.

Pour ce faire, on ajoute à la solution de polysaccharide ou mucopolyssacharide une solution d'acide périodique ou d'un de ses sels jusqu'à l'obtention d'une concentration comprise entre 0,01 et 1 M, de préférence 0,25 à 0,5 M.

L'étape d'oxydation peut être opérée sur des solutions, des gels ou des suspensions de polysaccharide(s).

La préparation de polysaccharide oxydé peut ensuite être soumise à des dialyses, diafiltrations, filtrations, ultrafiltrations dans le but d'éliminer les produits de la réaction d'oxydation et des réactifs, ainsi que des dérivés iodés formés pendant la réaction, ou en excès.

On peut également prévoir une lyophilisation, la redissolution du lyophilisat pouvant se faire en eau ou avec le tampon physiologique nécessaire.

La composition adhésive selon l'invention comprend au moins un polyaldéhyde macromoléculaire biodégradable d'origine naturelle tel que défini ci-dessus.

Il peut s'agir d'une solution aqueuse comprenant de préférence de 0,5 à 5 % en poids de polyaldéhyde(s).

On a constaté que la solution aqueuse de polyaldéhyde devient spontanément acide, ce qui favorise sa stabilité. En présence de tampons modifiant ce pH, on observe une perte de réactivité progressive dans le temps. On conserve donc avantageusement le polyaldéhyde au pH acide qu'il acquiert naturellement en solution aqueuse, jusqu'au moment de l'emploi ou encore, sous forme lyophilisée.

La solution est stable à l'abri de l'air et est conservée de préférence entre +1°C et +25°C.

Cette composition adhésive est avantageusement utilisée, après neutralisation par un tampon approprié avant l'emploi, pour la liaison de tissus biologiques entre eux ou à un biomatériau implanté.

Dans ces conditions, la solution de polyaldéhyde neutralisée est stable pendant au moins une journée à température ambiante.

La température d'application de la solution de polyaldéhyde est comprise entre 20 et 45°C.

Dans le cas d'un biomatériau implanté, celui-ci présente des fonctions réactives vis-à-vis du ou des polyaldéhydes contenus dans la composition qui sont formées en particulier par des fonctions aminées.

Le matériau peut comprendre du collagène ou de la gélatine comme décrit ci-après.

On prévoit avantageusement un temps de contact entre le biomatériau et la composition adhésive, de l'ordre de 10 secondes à 3 minutes préalablement à l'application "in situ" dudit matériau.

Le collage se produit progressivement en général entre 10 secondes et 3 minutes à pH physiologique, à une température de l'ordre de 37-40°C.

On peut contrôler ce temps de collage et le temps de dégradation in vivo de l'adhésif formé en fonction de la concentration de la solution de polyaldéhyde macromoléculaire ainsi que selon le taux d'oxydation du polysaccharide comme indiqué ci-après.

Selon un second mode de réalisation de l'invention, la composition adhésive comprend une solution contenant au moins un polyaldéhyde macromoléculaire biodégradable d'origine naturelle, telle que définie précédemment ainsi qu'une solution d'un composant collagénique.

Selon l'invention, le terme "composant collagénique" désigne du collagène ayant perdu au moins partiellement sa structure hélicoïdale par une opération de chauffage ou toute autre méthode, ou encore du collagène natif.

Dans le cas d'un chauffage pour dénaturer la structure hélicoïdale du collagène, le chauffage doit être modéré et effectué dans des conditions douces, de manière à éviter la dégradation par coupure hydrolytique, de la gélatine ainsi formée.

Le collagène est non hydrolysé et constitué en majorité de chaînes α.

Par chaînes α dans le sens de l'invention, on entend des chaînes α entières ou des fragments différents des chaînes α entières par la perte d'un petit nombre d'acides aminés.

Le poids moléculaire des chaînes α entières est généralement d'environ 100 kDa, c'est-à-dire de 95 à 130 kDa selon les cas.

Le terme "non hydrolysé" tel qu'utilisé selon l'invention signifie que moins de 10 % des chaînes collagéniques ont un poids moléculaire inférieur à environ 100 kDa.

Par "collagène natif", on entend du collagène qui a conservé sa structure hélicoïdale d'origine mais qui peut avoir été modifié chimiquement ou encore traité pour éliminer les télopeptides, comme indiqué ci-après.

Le collagène utilisé selon l'invention peut être d'origine humaine ou animale, de type I, III ou IV ou issu de leur mélange.

Le collagène de départ peut également être modifié chimiquement par méthylation, par succinylation ou tout autre méthode connue.

Le collagène est de préférence exempt de télopeptides, éliminés notamment par traitement à la pepsine de manière à le rendre filtrable sur des filtres de porosité adaptée à la stérilisation (élimination des microbes).

Les gélatines du commerce ne sont pas utilisables aux fins de l'invention. Des colles comprenant de telles gélatines donnent des gels qui ne deviennent fluides et utilisables, notamment en seringues, qu'à des températures élevées supérieures à 45-50°C, températures pouvant engendrer des réactions de brûlure et de nécrose des tissus (cerveau, nerf, intestin, cornée) alors que l'objectif est au contraire de les protéger.

Conformément à l'invention, le collagène est mis en oeuvre sous forme de solution aqueuse.

Pour cela, le collagène est solubilisé dans de l'eau dans des conditions stériles, en chauffant avantageusement à une température appropriée comprise entre 40 et 70°C.

Les conditions de solubilisation seront adaptées en fonction du collagène utilisé.

La préparation de gélatine ainsi obtenue peut être soumise ensuite à une filtration dans des conditions stériles, en maintenant la température entre 40 et 70°C.

La solution collagénique est avantageusement à pH physiologique, en présence ou non d'un tampon, notamment un tampon phosphate.

Selon l'invention, la solution de gélatine peut être une solution de 10 à 20 %, 15 % à 18 % de préférence.

Selon une variante de l'invention, notamment pour des préparations de gélatine à concentration élevée voisine de 20 %, difficilement filtrables, la solution peut être préparée dans des conditions stériles et utilisée pour obtenir directement une composition adhésive selon l'invention.

Selon cette variante, pour ces concentrations élevées, la préparation peut être obtenue par lyophilisation d'une solution diluée stérile puis redissolution de la poudre stérile par de l'eau stérile dans une enceinte stérile, ce qui permet de réaliser l'opération sans risque de contamination.

D'une manière générale, le collagène est chauffé au-dessus de 37°C et perd ainsi la majeure partie de sa structure hélicoïdale en triple hélice. On peut assimiler la préparation finale à de la gélatine mais dont le poids moléculaire des chaînes élémentaires est supérieur ou égal à 100 000 Daltons, avec moins de 10 % des chaînes de poids moléculaire inférieur. Cette gélatine est ainsi non hydrolysée et se distingue des gélatines couramment disponibles dans le commerce.

Selon l'invention, lorsqu'on utilise le terme "gélatine", on se réfère à une telle préparation.

Si l'on souhaite garder la structure hélicoïdale du collagène, notamment pour augmenter le temps de dégradation in vivo, la solution n'est pas chauffée. On préfère alors préparer des solutions moins concentrées, inférieures à 5 %, de préférence entre 0,2 et 5 % environ, notamment entre 1 et 3 % en partant de collagène natif éventuellement pepsiné.

Le procédé de réticulation selon l'invention comprend le fait de mélanger les deux solutions précitées. On réalise avantageusement un mélange homogène.

Le polyaldéhyde est mélangé au collagène ou gélatine selon des proportions de 0,5 à 10 % en poids, de préférence de 3 à 10 % en poids.

Le mélange des solutions précitées est opéré de préférence à pH neutre, physiologique, et à une température comprise entre 20 et 45°C, de préférence de l'ordre de 37 à 92°C environ.

Dans le cas de collagène natif utilisé selon une concentration inférieure à 5 %, on préfère mettre en oeuvre la solution collagénique à température ambiante, par exemple voisine de 20°C à 30°C.

La composition adhésive et le procédé de réticulation selon l'invention peuvent être mis en oeuvre notamment pour arrêter le saignement de plaies tissulaires, pour lier des tissus biologiques entre eux ou à un biomatériau implanté, pour la cicatrisation des plaies chirurgicales ou chroniques, pour la protection ou l'étanchéité des sutures, pour l'inhibition de la formation d'adhérences post-opératoires ou encore la délivrance de médicaments selon un système de libération prolongée, ainsi qu'en chirurgie réfractive de l'oeil, en paticulier l'épikératoplastie.

Selon les applications, le biomatériau implanté consiste en l'adhésif lui-même qui est alors utilisé seul.

L'invention trouve une application particulièrement avantageuse dans la prévention de la formation d'adhérences post-opératoires.

Elle permet également le collage de lentilles, en particulier de lentilles en collagène, sur la cornée dans le domaine de la chirurgie réfractive de l'oeil.

L'invention fournit ainsi un procédé de liaison d'un biomatériau à des tissus biologiques, comprenant le fait d'appliquer à une température comprise entre 20° et 45°C, un polyaldéhyde macromoléculaire biodégradable d'origine naturelle tel que défini précédemment, sous forme d'une solution aqueuse au pH acide naturellement obtenu ou neutralisé avant l'emploi, sur lesdits tissus et/ou ledit biomatériau, celui-ci présentant des fonctions, notamment aminées, réactives vis-à-vis dudit polyaldéhyde.

Selon un autre aspect, l'invention fournit un procédé de liaison de tissus biologiques entre eux ou à un biomatériau implanté, comprenant le fait de mélanger à pH neutre, physiologique, une solution aqueuse de polyaldéhyde macromoléculaire biodégradable d'origine naturelle, telle que décrit précédemment, avec une solution aqueuse de composant collagénique tel que défini précédemment.

On applique ensuite rapidement, c'est-à-dire de préférence en moins d'une minute, le gel ainsi obtenu sur lesdits tissus et/ou ledit biomatériau, celui-ci présentant des fonctions, notamment aminées, réactives vis-à-vis du polyaldéhyde macromoléculaire, à une température comprise entre 20° et 45°C, de préférence 37° à 42°C, puis on laisse polymériser l'ensemble.

Lorsque le composant collagénique est du collagène natif, il n'est pas nécessaire de chauffer la composition adhésive qui peut alors être appliquée à température ambiante.

Les solutions du composant collagénique et de polyaldéhyde peuvent résulter de la redissolution en milieu aqueux tamponné ou non, desdits composants conservés respectivement sous forme lyophilisée.

Par le mélange de la solution de composant collagénique et de la solution de polyaldéhyde macromoléculaire, le gel homogène et visqueux obtenu se prend en masse progressivement et durcit rapidement en adhérant fortement par exemple aux tissus biologiques sur lesquels il est appliqué.

Selon l'invention, on peut contrôler le temps de réticulation et l'augmenter au-delà de 30 secondes en diminuant la concentration de la solution de polyaldéhyde notamment en-dessous de 3 % pour une même proportion de collagène.

Par exemple, des concentrations de polyaldéhyde macromoléculaire comprises entre 3 et 0,5 % permettent de moduler le temps de polymérisation d'une solution de gélatine à 15 %, respectivement de 15 secondes à 5 minutes.

La polymérisation se produit progressivement en général à pH physiologique à une température de l'ordre de 37 à 40°C.

Selon une variante de l'invention, on peut également ajuster le temps de résorption in vivo de l'adhésif formé en modifiant le taux d'oxydation du polysaccharide mis en oeuvre pour l'obtention du polyaldéhyde macromoléculaire utilisé. Celui-ci peut ainsi être ajusté entre 1 à 2 jours et 30 à 60 jours.

A cette fin, on peut avantageusement faire varier les concentrations finales en périodate de sodium entre 0,05 M et 0,1 M.

D'une manière générale plus la concentration en polyaldéhyde est augmentée plus le temps de résorption augmente également.

L'invention fournit également des kits destinés à un usage chirurgical ou thérapeutique notamment pour les applications citées précédemment.

Selon une première forme de réalisation, le kit comprend une solution contenant au moins un polyaldéhyde macromoléculaire biorésorbable placée dans une seringue.

On emploie de préférence une solution aqueuse acide contenant 0,5 à 5 % en poids de polyaldéhyde(s).

Selon une autre forme de réalisation de l'invention, le kit peut se présenter avantageusement sous la forme de deux seringues dont chacune comprend respectivement la solution de composant collagénique et la solution de polyaldéhyde macromoléculaire. La solution de collagène est, dans sa propre seringue, à un pH physiologique, avec ou sans tampon (notamment un tampon phosphate).

Les seringues sont fixées à un dispositif de maintien équipé de moyens de mélange conçus pour pouvoir mélanger extemporanément leur contenu de manière homogène, après les avoir réchauffées à la température appropriée comprise entre 37 et 45°C, selon la fluidité souhaitée.

Le kit peut comprendre une solution de 10 à 20 % de collagène non hydrolysé, constitué majoritairement de chaînes α, et une solution de polyaldéhyde de 0,5 à 5 %.

Selon un mode de réalisation préférentiel, on prévoit un kit comprenant d'une part du collagène en solution à 18 % et, d'autre part, de l'amidon oxydé à 3 %, dans un rapport de 1/24.

Si l'on utilise du collagène natif, le kit comprend une solution aqueuse à 5 % ou moins, de préférence selon une concentration de 0,2 à 5 %, plus préférentiellement 1 à 3%.

La composition adhésive selon l'invention, et notamment le kit décrit ci-dessus, présente l'avantage de pouvoir être stockée entre + 1 et + 25°C, de préférence entre +2 et +8° C.

D'autre part, selon l'invention le temps de réticulation peut être raccourci en dessous de 3 minutes et contrôlé selon les exigences requises.

Parallèlement, la force de collage développée par le produit selon l'invention est supérieure à celle obtenue avec les colles connues, et notamment les colles de fibrine, tout en conservant une excellente biocompatibilité.

En outre, l'invention offre avantageusement la sécurité d'emploi liée aux méthodes de préparation du collagène efficaces pour l'élimination des virus et prions (Agents Transmissibles Non Conventionnels) notamment par l'emploi d'un traitement acalin. Ceci écarte un des problèmes majeurs soulevés par les colles de fibrine.

De plus, par rapport à ces colles de fibrine, la viscosité de la composition adhésive selon l'invention est supérieure tout en restant en-dessous de 1000 centipoises, et facilite ainsi son adhésion immédiate.

L'invention va être décrite plus en détails à l'aide des exemples donnés ci-après à titre indicatif et non limitatif.

### Exemple 1 : Réalisation d'une solution de polyaldéhyde macromoléculaire.

On ajoute à une solution d'Amidon soluble purifié ou de Dextran présentant ou non des charges positives (groupements aminés) ou négatives (groupements carboxyliques ou sulfoniques) ou tout autre groupement fonctionnel, dont le poids moléculaire peut varier de 10 000 à 2 millions Daltons, à une concentration de 5 % en eau, une solution d'acide périodique ou de périodate de sodium jusqu'à l'obtention d'une concentration comprise entre 0,01 et 1M, de préférence 0,25 M.

Après 2 heures de contact à la température du laboratoire, la solution est dialysée (ou diafiltrée à l'aide d'un ultrafiltre) avec une membrane de seuil de coupure 5 000 Daltons, contre de l'eau distillée. La dialyse est poursuivie jusqu'à l'élimination totale des produits dialysables de la réaction d'oxydation et des réactifs, ainsi que des dérivés iodés formés pendant la réaction, ou en excès.

On a constaté que la solution de polyaldéhyde obtenue devient spontanément acide, ce qui favorise sa stabilité, et on préfère donc ne pas ajouter de tampon neutre.

La solution finale concentrée de polymère polyaldéhydique dérivé du polysaccharide initial est ensuite saturée en azote par bullage et conditionnée stérilement par filtration sur membrane de porosité 0,2 µ.

Le produit est stable pendant au moins un an entre +4°C et 25°C, à l'abri de l'air.

Pour la réalisation d'un kit selon l'invention, on conditionne la solution préparée en seringues.

### Exemple 2 : Réalisation d'une solution de collagène.

On prépare une solution acide de collagène à 15 % par dissolution d'une poudre de collagène acide dans l'eau, à une température comprise entre 40°C et 70°C pendant 30 minutes.

La solution est neutralisée à pH 7,5 par addition de soude normale dès que la fluidité le permet.

Dans le cas du collagène bovin de type I, il peut être acidosoluble (extrait de dermes et de tendons à pH acide), ou solubilisé par digestion à la pepsine, ce qui le rend plus facile à filtrer par la suite.

Dans le cas des collagènes humains d'origine placentaire, ils peuvent être préparés par extraction à la pepsine selon le procédé décrit dans la demande EP-A-0 214 035.

Bien entendu, d'autres sources de collagène peuvent être utilisées parmi celles connues par l'homme du métier.

Puis la solution de collagène ainsi chauffée, fluidifiée et neutralisée, est filtrée stérilement sur membrane de porosité 0,2 µ à la suite de préfiltrations sur filtres de porosité progressivement décroissante, à une température appropriée entre 40°C et 70°C.

Si la filtration à une concentration en collagène de 20 % (ou plus) ne peut être réalisée, on dilue la solution collagénique préalablement à sa filtration.

Selon une variante, on lyophilise la solution diluée stérile puis on redissout la poudre stérile par de l'eau stérile dans une enceinte stérile à la concentration désirée.

Le produit est stable pendant au moins un an à température ambiante.

Pour la réalisation d'un kit selon l'invention, la solution de collagène ainsi obtenue peut être aisément répartie en seringues à température de 40°C.

### Exemple 3 : Réalisation d'un mélange adhésif.

On prépare une seringue de 0,5 ml de polyaldéhyde macromoléculaire à 5 % selon l'exemple 1, et une seringue de 2 ml de collagène à 15 %, selon l'exemple 2. Chacune des seringues est fixée à un dispositif de maintien équipé d'un mélangeur conçu pour pouvoir mélanger leur contenu respectif de manière homogène après les avoir réchauffées à une température de 40°C à 42°C, voire 45°C si une plus grande fluidité est souhaitée.

Le gel homogène et visqueux obtenu en sortie se prend en masse à cette même température, progressivement à partir de 15 à 30 secondes à pH physiologique et durcit rapidement en adhérant fortement aux tissus biologiques sur lesquels il est appliqué.

Si le mélange est appliqué sur une surface tissulaire ou sur une plaie dont la température est inférieure à 37°C, la polymérisation induite par la réaction chimique entre les fonctions aldéhydes et les fonctions amines du collagène, est complétée par une solidification résultant de la gélification du collagène en dessous de 37°C.

Le temps de polymérisation peut être augmenté au delà de 15 secondes par dilution du polyaldéhyde macromoléculaire en dessous de 5 % ou en utilisant une quantité par seringue inférieure à 0,5 ml pour 2 ml de collagène.

Des concentrations de polyaldéhyde macromoléculaire comprises entre 5 et 0,5 % permettent de moduler le temps de polymérisation, respectivement de 15 secondes à 5 minutes.

Il est possible aussi d'utiliser une solution à 5 % de polysaccharide moins oxydé, préparé par exemple avec une concentration finale de périodate de sodium comprise entre 0,05 M et 0,1 M, en variante de l'exemple 1.

### TEST D'ADHESIVITE

On mesure la force développée par l'adhésif tissulaire ainsi préparé, en appliquant le mélange décrit ci-dessus entre deux lambeaux de peau de porc de 6,25 cm², eux-mêmes reliés séparément par collage avec une colle acrylique du côté épidermique sur deux plateaux d'un dynamomètre.

Après avoir appliqué le produit mélangé et fluide à 40°C sur chacun des deux lambeaux sur la face dermique, les deux plateaux sont aussitôt rapprochés pour comprimer le tout avec une force de 4 Newtons pendant 1 minute puis on incube à 37°C pendant 30 minutes.

Lorsque les plateaux sont séparés progressivement, on mesure la résistance à la rupture des deux lambeaux.

La force mesurée est de 7,26 Newtons. Le travail fourni pour lutter contre la résistance à l'étirement est de 10,96 millijoules.

Dans les mêmes conditions, la colle de fibrine "TISSUCOL" donne une force de 6,30 Newtons et de 11,25 millijoules.

### Exemple 4 :

Les force ou travail de l'exemple 3 peuvent être augmentés du double ou du triple en augmentant la concentration du collagène ou du polyaldéhyde macromoléculaire, comme illustré ci-après :

On prépare une seringue de 0,5 ml d'une solution de polyaldéhyde macromoléculaire à 5 % selon l'exemple 1, et une seringue de 2 ml de collagène à 20 %, selon une variante de l'exemple 2.

On procède comme à l'exemple 3.

Dans le test d'adhésivité, la force mesurée est de 12,15 Newtons, et le travail fourni pour lutter contre la résistance à l'étirement est de 27,72 millijoules.

### Exemple 5 : Collage d'une lentille en collagène avec une solution de polyaldéhyde macromoléculaire sur une cornée de lapin et sur une cornée de primate non humain.

Pour réaliser le collage d'une lentille en collagène, on maintient les paupières ouvertes avec un blépharostat, on désépithélialise la cornée sur une zone plus large que le diamètre de la lentille, puis on lave soigneusement la zone désépithélialisée pour éliminer les débris cellulaires.

On dépose la solution de polyaldéhyde à 2,5 % ou 5 % préparée selon la procédure de l'exemple 1, dans la face concave de la lentille jusqu'à remplir complètement la concavité de celle-ci avec la solution de polyaldéhyde.

Après 30 secondes à 2 minutes de contact, on sèche la surface cornéenne, on élimine l'excédent de solution de polyaldéhyde par aspiration, puis on place la face concave de la lentille sur la cornée, en prenant soin de centrer la lentille par rapport à l'axe optique.

Le bord de la lentille est correctement plaqué contre la cornée à l'aide d'une spatule.

Après 5 minutes de contact, il est possible de mobiliser le globe oculaire par l'intermédiaire d'une spatule posée sur la lentille.

Dans ces conditions, l'utilisation de polyaldéhyde macromoléculaire à une concentration comprise entre 2,5 et 5 %, n'induit pas de réaction inflammatoire clinique sur les structures antérieures du globe oculaire : cornée, conjonctive, iris, paupières, ni chez le lapin albinos (New Zealand) ni chez le primate non humain (Macaccus cynomolgus).

Cette absence de réactions toxiques ou d'irritation locales souligne l'avantage de la composition adhésive selon l'invention par rapport à l'utilisation des agents réticulants traditionnels de l'art antérieur, pour la réalisation d'adhésifs tissulaires.

### Exemple 6 : Prévention de la formation d'adhérences post-opératoires.

Le modèle expérimental utilisé pour démontrer les propriétés de l'adhésif selon l'invention dans la prévention d'adhérences postopératoires a été publié par Elisabeth HARRIS et l'équipe de George RODEHEAVER (Surgery, 117, 6, 663-669, 1995).

Le protocole décrit dans cette publication a été mis en oeuvre sur des groupes de 10 rats.

Les essais consistent à créer une abrasion et une déshydratation sur des surfaces de 2 cm² de paroi péritonéale et de caecum, au contact l'une de l'autre.

Le groupe de rats témoins ne reçoit aucun produit de protection des plaies ainsi crées ; il est comparé au groupe de rats qui reçoit de 1 ml à 2 ml de l'adhésif tissulaire de l'invention selon l'exemple 3, en utilisant un kit comprenant deux seringues comme décrit dans cet exemple, l'adhésif étant appliqué sur chacune des deux plaies en regard l'une de l'autre.

Après 7 jours d'attente, conformément au protocole publié, les résultats sont nets :

Aucune adhérence n'est observée entre les deux surfaces lésées dans le groupe de rats traités par l'adhésif tissulaire selon l'invention.

Le groupe de rats témoins, non traité par l'adhésif tissulaire de l'invention, montre des adhérences pour chacun des 10 rats, dont les caractéristiques sont identiques aux résultats publiés dans Surgery 117, 6, 663-669, 1995 mentionné ci-dessus.

Il ressort de cette même publication (table II, page 667), que la colle de fibrine (Fibrin sealant), n'est pas efficace pour inhiber complètement la formation d'adhérences post-opératoires.

Les propriétés anti-adhérences démontrées pour l'adhésif tissulaire de cette invention sont particulièrement surprenantes quand on les compare à celles des colles de fibrine qui n'ont pas ces propriétés.

Selon l'invention, on peut appliquer la composition adhésive formant barrière anti-adhérences en un endroit précis, y compris par injection à travers un cathéter, sans risque de voir bouger cette barrière, ce qui renforce la sécurité et l'efficacité pour le patient traité.

### Exemple 7 :Influence de la concentration en polyaldéhyde macromoléculaire sur le temps de réactivité de la composition adhésive selon l'invention

On prépare une composition adhésive à base d'une part, de collagène à 16,5 % et, d'autre part, d'amidon oxydé sous forme liquide pH 3,3, à 3,3% (concentration moyenne déterminée par mesure de l'indice de réfraction (3,1 %) et par contrôle des matières sèches (3,5 %)).

Dans cette étude, on procède à des dilutions de l'amidon oxydé, dans l'eau, à 1 %, 0,75 % et 0,5 % respectivement.

Par ailleurs, on part de solutions de collagène à des pH différents, en présence de tampon phosphate.

Les résultats obtenus sont indiqués dans le tableau 1 ci-dessous :

**TABLEAU 1**

| pH collagène | concentration en phosphate (M) | concentration de l'amidon oxydé (concentration finale) | temps de polymérisation du mélange |
|---|---|---|---|
| 7,44 | 0,05 | 1 % (0,2 %) | 45 sec. |
| | | 0,75 % (0,15 %) | 1 min. et 5 sec. |
| | | 0,5 % (0,1 %) | 3 min. |
| 7,08 | 0,02 | 1 % (0,2 %) | 2 min. |
| | | 0,75 % (0,15 %) | 2 min. et 30 sec. |
| | | 0,5 % (0,1 %) | 5 min. |
| 7,33 | 0,02 | 1 % (0,2 %) | 1 min. et 15 sec. |
| | | 0,75 % (0,15 %) | 2 min. |
| | | 0,5 % (0,1 %) | 4 min. |

Ces résultats montrent que le mélange adhésif durcit d'autant plus rapidement que la concentration en amidon oxydé et le pH du mélange sont élevés.

Les concentrations en amidon oxydé de 1 % et 0,75 % conduisent à des résultats proches, à 30 secondes près. Par contre, on note une différence sensible entre les concentrations 0,75 % et 0,5 %.

L'ensemble des gels présentent une bonne cohésion et ils ont un aspect élastique d'autant plus prononcé que la concentration en amidon oxydé est faible, cette différence étant durable dans le temps.

Le gel avec l'amidon oxydé à 0,5 % présente une grande élasticité.

La concentration standard d'amidon oxydé à 3% conduit, quant à elle, à un gel très résistant et d'élasticité plus faible. Le gel augmente son élasticité avec des concentrations en amidon oxydé de l'ordre de 1 % ou moins. Cette propriété peut être intéressante dans de nombreuses applications.

### Exemple 8 : Influence de la concentration en tampon et du pH du collagène sur le temps de réactivité de la composition adhésive selon l'invention

On prépare une composition adhésive à base, d'une part, de collagène à 17 % environ et, d'autre part, d'amidon oxydé forme liquide pH 3,3, à 3,3% comme à l'exemple 7.

On ajoute un tampon phosphate au collagène selon des concentrations variant de 0,01M à 0,1M et l'on prévoit un témoin collagène sans phosphate, ajusté aux mêmes pH.

On étudie parallèlement l'influence du pH du collagène en préparant des solutions à pH variant de 6,9 à 7,45.

Les résultats obtenus sont indiqués dans le tableau 2 ci-dessous.

**TABLEAU 2**

| Concentration en collagène (%) | pH collagène | Concentration phosphate M | Temps de polymérisation du mélange | pH après mélange avec l'amidon oxydé |
|---|---|---|---|---|
| 17 | 7,12 | 0 | 45 sec. | 6,75 |
| 17,3 | 7,44 | 0,05 | 18 sec. | 7,15 |
| 17 | 7,45 | 0.1 | 15 sec. | 7,30 |
| 16,6 | 7,05 | 0 | 1 min. et 20 sec. | 6,60 |
| 16,8 | 7,25 | 0 | 1 min. et 05 sec. | 6,69 |
| 16,9 | 7,06 | 0,05 | 33 sec. | 6,94 |
| 16,9 | 7,36 | 0,05 | 17 sec. | 7,11 |
| 16,6 | 6,95 | 0,01 | 1 min. | 6,72 |
| 16,7 | 7,08 | 0,02 | 40 sec. | 6,92 |
| 17 | 7,33 | 0,02 | 28 sec. | 7,02 |

On constate que la réactivité du mélange augmente avec le pH du collagène et son pouvoir tampon.

### Exemple 9 : Réalisation d'un mélange adhésif à partir de collagène natif

On utilise du collagène solubilisé par digestion à la pepsine et purifié, en solution aqueuse contenant 9 g/l de chlorure de sodium ajusté à pH 7, selon une concentration de 2 %.

Le collagène non modifié chimiquement ou modifié par succinylation ou méthylation peut être utilisé indifféremment dans cet exemple. Dans tous les cas, il a conservé sa structure hélicoïdale.

Le produit est réparti stérilement dans une seringue de 2 ml stockée à +4°C.

Une solution d'amidon oxydé à 1 % est préparée en dissolvant la poudre acide d'amidon oxydé lyophilisée par un tampon phosphate 0,2M pH 7,50. Une seringue de 0,5 ml est préparée.

Les deux seringues de collagène et d'amidon sont réunies dans un kit semblable à l'exemple 3, réchauffé à température ambiante (voisine de 20°C).

Pour évaluer la force d'adhésion du mélange obtenu à partir de ce kit, on réalise le test d'adhésivité selon l'exemple 3 avec des lambeaux de peau de porc de 6,25 cm².

Les résultats obtenus sont les suivants :

La force mesurée est de 4,5 Newtons. Le travail fourni pour résister à l'étirement est de 3,15 millijoules.

## Revendications

1. Composition adhésive biocompatible, biorésorbable et non toxique, à usage chirurgical et/ou thérapeutique, notamment pour la liaison de tissus biologiques entre eux ou à un biomatériau implanté, comprenant au moins un polysaccharide ou mucopolysaccharide oxydé polyaldéhydique macromoléculaire biodégradable d'origine naturelle, en solution aqueuse, **caractérisée en ce qu'**elle comprend au moins un polyaldéhyde macromoléculaire biodégradable d'origine naturelle en solution aqueuse au pH acide naturellement obtenu.

2. Composition adhésive biocompatible, biorésorbable et non toxique, à usage chirurgical et/ou thérapeutique, notamment pour la liaison de tissus biologiques entre eux ou à un biomatériau implanté, **caractérisée en ce qu'**elle comprend :
au moins un polysaccharide ou mucopolysaccharide oxydé polyaldéhydique macromoléculaire biodégradable d'origine naturelle,
et un composant collagénique, solubilisé en milieu aqueux, choisi parmi
- le collagène ayant perdu au moins partiellement sa structure hélicoïdale, non hydrolysé, constitué majoritairement de chaîne α, ladite composition ne requérant alors, lors de l'utilisation finale, qu'un chauffage permettant la préparation de la composition adhésive par mélange, puis son application sur les tissus à une température comprise entre 20°C et 45°C,
- le collagène natif selon une concentration inférieure à 5%, ladite composition permettant, alors, l'utilisation finale à la température ambiante.

3. Composition adhésive selon la revendication 1 ou 2, **caractérisée en ce qu'**elle comprend au moins un polyaldéhyde macromoléculaire biodégradable d'origine naturelle en solution aqueuse au pH acide naturellement obtenu.

4. Composition adhésive selon l'une quelconque des revendications précédentes, **caractérisée en ce que** le polysaccharide ou mucopolysaccharide est choisi parmi le groupe comprenant l'amidon, le dextrane, l'agarose, la cellulose, la chitine, le chitosan, l'acide alginique, les glycosaminoglycanes, l'acide hyaluronique et le chondroïtine sulfate ou leurs dérivés.

5. Composition adhésive selon l'une quelconque des revendications précédentes, **caractérisée en ce que** le polysaccharide est choisi parmi l'amidon et le dextrane et est de préférence l'amidon.

6. Composition adhésive selon l'une quelconque des revendications précédentes, **caractérisée en ce que** le polyaldéhyde consiste en un polysaccharide ou mucopolysaccharide oxydé par l'acide périodique ou l'un de ses sels.

7. Composition adhésive selon l'une quelconque des revendications précédentes, **caractérisée en ce que** le polysaccharide présente un poids moléculaire compris entre environ 10 000 et 2 millions Daltons.

8. Composition adhésive selon l'une quelconque des revendications précédentes, **caractérisée en ce qu'**elle comprend une solution aqueuse de polyaldéhyde(s) selon une concentration de 0,5 à 5% en poids.

9. Composition adhésive selon l'une quelconque des revendications précédentes, **caractérisée en ce que** la solution aqueuse est obtenue par dissolution dans l'eau ou avec un tampon physiologique, d'un polyaldéhyde sous forme lyophilisée acide.

10. Composition adhésive selon l'une quelconque des revendications 2 à 9, **caractérisée en ce qu'**elle comprend une solution de collagène ayant perdu au moins partiellement sa structure hélicoïdale, non hydrolysé, selon une concentration de 10 à 20%, de préférence 15 à 18%.

11. Composition adhésive selon l'une quelconque des revendications 2 à 9, **caractérisée en ce qu'**elle comprend une solution aqueuse de collagène natif selon une concentration de 0,2 à 5%, de préférence 1 à 3%.

12. Composition adhésive selon l'une quelconque des revendications 2 à 11, **caractérisée en ce que**, pour le mélange, la proportion du polyaldéhyde par rapport au collagène est de 0,5 à 10% en poids, de préférence 3 à 10%.

13. Composition adhésive selon l'une quelconque des revendications précédentes, **caractérisée en ce qu'**elle est résorbable dans l'organisme entre 1 à 2 jours et 30 à 60 jours.

14. Procédé de préparation d'une colle biocompatible, biorésorbable et non toxique, à usage chirurgical et/ou thérapeutique, destinée à être appliquée sur des tissus et/ou un biomatériau, **caractérisé en ce qu'**il comprend l'étape consistant à mélanger à un composant collagénique, solubilisé en milieu aqueux, choisi parmi
- le collagène ayant perdu au moins partiellement sa structure hélicoïdale, non hydrolysé, constitué essentiellement de chaînes α,
- le collagène natif selon une concentration inférieure à 5%, préalablement à sa réticulation, au moins un polysaccharide ou mucopolysaccharide oxydé polyaldéhydique macromoléculaire biodégradable d'origine naturelle, en solution aqueuse.

15. Procédé selon la revendication 14, **caractérisé en ce que** la solution de composant collagénique et la solution de polyaldéhyde macromoléculaire sont telles que définies selon l'une quelconque des revendications 3 à 13.

16. Procédé selon l'une quelconque des revendications 14 et 15, **caractérisé en ce que** l'on réalise le mélange à une température comprise entre 20 et 45°C.

17. Procédé selon la revendication 16, **caractérisé en ce que** l'on réalise le mélange à une température de l'ordre de 37 à 42°C.

18. Procédé selon l'une quelconque des revendications 14 à 17, **caractérisé en ce que** l'on réalise le mélange à un pH neutre, physiologique.

19. Procédé selon l'une quelconque des revendications 14 à 18, **caractérisé en ce que** la réticulation est réalisée en moins de 5 minutes.

20. Utilisation d'un polysaccharide ou mucopolysaccharide oxydé polyaldéhydique macromoléculaire biodégradable d'origine naturelle, en solution aqueuse, pour l'obtention d'une composition adhésive biocompatible, biorésorbable et non toxique, à usage chirurgical et/ou thérapeutique pour la liaison de tissus biologiques entre eux ou à un biomatériau implanté présentant des fonctions réactives aminées vis-à-vis dudit polyaldéhyde.

21. Utilisation selon la revendication 20, en combinaison avec un composant collagénique, solubilisé en milieu aqueux, choisi parmi
- le collagène ayant perdu au moins partiellement sa structure hélicoïdale, non hydrolysé, constitué majoritairement de chaînes α,
- le collagène natif selon une concentration inférieure à 5%.

22. Utilisation selon l'une quelconque des revendications 20 et 21, pour l'obtention d'une composition adhésive telle que définie selon l'une quelconque des revendications 1 à 13.

23. Kit comprenant une composition selon la revendication 1.

24. Kit selon la revendication 23, **caractérisé en ce qu'**il se présente sous la forme d'une seringue.

25. Kit selon la revendication 23 ou 24, **caractérisé en ce qu'**il comprend une solution aqueuse de polyaldéhyde(s) selon une concentration comprise entre 0,5 et 5% en poids.

26. Kit selon la revendication 23, **caractérisé en ce qu'**il comprend en outre :
- une solution aqueuse d'un composant collagénique choisi parmi
. le collagène ayant perdu au moins partiellement sa structure hélicoïdale, non hydrolysé, constitué majoritairement de chaînes α,
. le collagène natif selon une concentration inférieure à 5%,
- des moyens de mélange pour mélanger extemporanément lesdites solutions.

27. Kit selon la revendication 26, **caractérisé en ce qu'**il se présente sous la forme de deux seringues équipées de moyens de mélange dont l'une des seringues contient la solution de composant collagénique et l'autre contient la solution de polyaldéhyde macromoléculaire.

28. Kit selon l'une quelconque des revendications 26 et 27, **caractérisé en ce qu'**il comprend une solution de polyaldéhyde de 0,5 à 5%.

29. Kit selon l'une quelconque des revendications 23 à 28, **caractérisé en ce que** la solution de polyaldéhyde est obtenue par dissolution dans l'eau ou avec un tampon physiologique, d'un polyaldéhyde sous forme lyophilisée acide.

30. Kit selon l'une quelconque des revendications 26 à 29, **caractérisé en ce qu'**il comprend une solution de collagène ayant perdu au moins partiellement sa structure hélicoïdale, non hydrolysé, constitué majoritairement de chaînes α, de 10 à 20%, de préférence 15-18%.

31. Kit selon l'une des revendications 26 à 30, **caractérisé en ce qu'**il comprend d'une part du collagène ayant perdu au moins partiellement sa structure hélicoïdale, non hydrolysé, constitué majoritairement de chaînes α, en solution aqueuse à 18% et, d'autre part, de l'amidon oxydé en solution aqueuse à 3%, selon un rapport 1/24.

32. Kit selon l'une quelconque des revendications 26 à 29, **caractérisé en ce qu'**il comprend une solution aqueuse de collagène natif selon une concentration de 0,2 à 5%, de préférence 1 à 3%.

33. Kit selon l'une quelconque des revendications 26 à 32, **caractérisé en ce que** la ou les solutions sont maintenues à une température comprise entre +1 et 25°C, de préférence entre +2°C et +8°C.

34. Kit selon l'une quelconque des revendications 23 à 33, **caractérisé en ce qu'**il comprend une composition selon l'une quelconque des revendications 1 à 13.

35. Utilisation selon la revendication 20 pour une composition destinée à être appliquée à une température comprise entre 20 et 45°C, sur lesdits tissus et/ou sur ledit biomatériau, ledit biomatériau présentant des fonctions, notamment aminées, réactives vis-à-vis dudit polyaldéhyde.

36. Utilisation selon la revendication 35, pour la liaison d'une lentille en collagène.

37. Utilisation selon la revendication 21 pour une composition que :
- l'on mélange à pH neutre, physiologique, une solution aqueuse d'un composant collagénique choisi parmi
. le collagène ayant perdu au moins partiellement sa structure hélicoïdale, non hydrolysé, constitué majoritairement de chaînes α,
. le collagène natif selon une concentration inférieure à 5% ;
et une solution aqueuse contenant au moins un polyaldéhyde macromoléculaire biodégradable d'origine naturelle;
- le gel obtenu étant appliqué rapidement sur lesdits tissus et/ou ledit biomatériau, celui-ci présentant des fonctions, notamment aminées, réactives vis-à-vis desdits polyaldéhydes macromoléculaires, à une température comprise entre 20 et 45°C ; et
- on laisse polymériser ledit mélange.

38. Utilisation selon l'une quelconque des revendications 35 à 37, **caractérisée en ce qu'**on utilise une solution aqueuse de polyaldéhyde au pH acide naturellement obtenu.

39. Utilisation selon l'une quelconque des revendications 35 à 37, **caractérisée en ce qu'**on utilise une solution aqueuse de polyaldéhyde au pH préalablement neutralisé.

40. Utilisation selon l'une quelconque des revendications 37 à 39, **caractérisée en ce que**, dans le cas de collagène ayant perdu au moins partiellement sa structure hélicoïdale, non hydrolysé, constitué majoritairement de chaînes α, on procède à une application à une température comprise entre 37 et 42°C.

41. Utilisation selon l'une quelconque des revendications 37 à 39, **caractérisée en ce que**, dans le cas de collagène natif, on procède à une application à la température ambiante.

42. Utilisation selon l'une quelconque des revendications 35 à 41, **caractérisée en ce que** l'on utilise une composition selon l'une quelconque des revendications 1 à 13.

43. Utilisation selon l'une quelconque des revendications 36 à 42, **caractérisée en ce que** l'on utilise un kit selon l'une des revendications 23 à 35.

44. Utilisation selon l'une quelconque des revendications 36 à 43, pour la prévention des adhérences post-opératoires.

## Claims

1. Non-toxic, biologically absorbable, biologically compatible, adhesive composition for surgical and/or therapeutic use, particularly for bonding biological tissues to one another or to an implanted biological material, comprising at least one natural, biodegradable, macromolecular, polyaldehydic, oxidized polysaccharide or mucopolysaccharide in an aqueous solution, **characterized in that** it comprises at least one biodegradable, macromolecular polyaldehyde having a natural origin in an aqueous solution and at a naturally obtained acid pH.

2. Non-toxic, biologically absorbable, biologically compatible, adhesive composition for surgical and/or therapeutic use, particularly for bonding biological tissues to one another or to an implanted biological material, **characterized in that** it comprises:
at least one natural, biodegradable, macromolecular, polyaldehydic, oxidized, polysaccharide or mucopolysaccharide,
and a collagenic component, solubilized in an aqueous medium and chosen from among
- collagen which has at least partly lost its helical structure and in non-hydrolyzed form, largely constituted by α chains, said composition then only requiring during the final use a heating permitting the preparation of the adhesive composition by mixing, followed by the application thereof to the tissues at a temperature between 20 and 45°C,
- native collagen in a concentration below 5%, said composition then permitting final use at ambient temperature.

3. Adhesive composition according to claim 2, **characterized in that** it comprises at least one biodegradable, macromolecular polyaldehyde having a natural origin in an aqueous solution and at a naturally obtained acid pH.

4. Adhesive composition according to any one of the preceding claims, **characterized in that** the polysaccharide or mucopolysaccharide is chosen from within the group including starch, dextran, agarose, cellulose, chitin, chitosan, alginic acid, glycosaminoglycans, hyaluronic acid and chondroitin sulphate or derivatives thereof.

5. Adhesive composition according to any one of the preceding claims, **characterized in that** the polysaccharide is chosen from among starch and dextran and is preferably starch.

6. Adhesive composition according to any one of the preceding claims, **characterized in that** the polyaldehyde consists of a polysaccharide or mucopolysaccharide oxidized by periodic acid or one of its salts.

7. Adhesive composition according to any one of the preceding claims, **characterized in that** the polysaccharide has a molecular weight between approximately 10,000 and 2 million Daltons.

8. Adhesive composition according to any one of the preceding claims, **characterized in that** it comprises an aqueous polyaldehyde solution in a concentration of 0.5 to 5 wt.%.

9. Adhesive composition according to any one of the preceding claims, **characterized in that** the aqueous solution is obtained by dissolving in water or with a physiological buffer a polyaldehyde in acid lyophilized form.

10. Adhesive composition according to any one of the claims 2 to 9, **characterized in that** it comprises a collagen solution having lost at least partly its helical structure and in non-hydrolyzed form, in a concentration of 10 to 20%, preferably 15 to 18%.

11. Adhesive composition according to any one of the claims 2 to 9, **characterized in that** it comprises an aqueous native collagen solution in a concentration of 0.2 to 5%, preferably 1 to 3%.

12. Adhesive composition according to one of the claims 2 to 11, **characterized in that,** for the mixture, the polyaldehyde proportion compared with the collagen proportion is 0.5 to 10 wt.%, preferably 3 to 10 wt.%.

13. Adhesive composition according to any one of the preceding claims, **characterized in that** it can be absorbed in the organism in between 1 and 2 days and 30 and 60 days.

14. Process for the preparation of a non-toxic, biologically absorbable, biologically compatible adhesive for surgical and/or therapeutic use and intended for application to tissues and/or a biological material, **characterized in that** it comprises the stage consisting of mixing with a collagenic component, solubilized in an aqueous medium, chosen from among
- collagen having at least partly lost its helical structure and non-hydrolyzed, essentially constituted by α chains,
- native collagen in a concentration below 5%, prior to its crosslinking, at least one natural, biodegradable, macromolecular, polyaldehydic, oxidized, polysaccharide or mucopolysaccharide in aqueous solution.

15. Process according to claim 14, **characterized in that** the collagenic component solution and the macromolecular polyaldehyde solution are as defined in any one of the claims 3 to 13.

16. Process according to either of the claims 14 and 15, **characterized in that** mixing takes place at a temperature between 20 and 45°C.

17. Process according to claim 16, **characterized in that** mixing takes place at a temperature of approximately 37 to 42°C.

18. Process according to any one of the claims 14 to 17, **characterized in that** mixing takes place at a physiological, neutral pH.

19. Process according to any one of the claims 14 to 18, **characterized in that** crosslinking takes place in less than 5 minutes.

20. Use of a natural, biodegradable, macromolecular, polyaldehydic, oxidized, polysaccharide or mucopolysaccharide in aqueous solution for obtaining a non-toxic, biologically absorbable, biologically compatible, adhesive composition for surgical and/or therapeutic use, for bonding biological tissues to one another or to an implanted material having reactive amino functions with respect to said polyaldehyde.

21. Use according to claim 20, in combination with a collagenic component solubilized in an aqueous medium and chosen from among
- collagen having at least partly lost its helical structure and non-hydrolyzed, largely constituted by α chains,
- native collagen with a concentration below 5%.

22. Use according to either of the claims 20 and 21 for obtaining an adhesive composition as defined in any one of the claims 1 to 13.

23. Kit comprising a composition according to claim 1.

24. Kit according to claim 23, **characterized in that** it is in the form of a syringe.

25. Kit according to claim 23 or 24, **characterized in that** it comprises an aqueous polyaldehyde solution in a concentration between 0.5 and 5 wt.%.

26. Kit according to claim 23, **characterized in that** it also comprises:
- an aqueous solution of a collagenic component chosen from among
• collagen having at least partly lost its helical structure and non-hydrolyzed, largely constituted by α chains,
• native collagen in a concentration below 5%,
- mixing means for mixing just prior to use the said solutions.

27. Kit according to claim 26, **characterized in that** it is in the form of two syringes equipped with mixing means, one of the syringes containing the collagenic component solution and the other containing the macromolecular polyaldehyde solution.

28. Kit according to either of the claims 26 and 27, **characterized in that** it comprises a 0.5 to 5% polyaldehyde solution.

29. Kit according to any one of the claims 23 to 28, **characterized in that** the polyaldehyde solution is obtained by dissolving in water or with a physiological buffer a polyaldehyde in acid lyophilized form.

30. Kit according to any one of the claims 26 to 29, **characterized in that** it comprises a collagen solution having at least partly lost its helical structure and non-hydrolyzed, largely constituted by a chains and 10 to 20% and preferably 15 to 18%.

31. Kit according to any one of the claims 26 to 30, **characterized in that** it comprises on the one hand collagen having at least partly lost its helical structure and non-hydrolyzed, largely constituted by α chains and in an 18% aqueous solution and on the other oxidized starch in a 3% aqueous solution and in a ratio of 1:24.

32. Kit according to any one of the claims 26 to 29, **characterized in that** it comprises an aqueous native collagen solution in a concentration of 0.2 to 5%, preferably 1 to 3%.

33. Kit according to any one of the claims 26 to 32, **characterized in that** the solution or solutions are kept at a temperature between +1 and 25°C, preferably between +2 and +8°C.

34. Kit according to any one of the claims 23 to 33, **characterized in that** it comprises a composition according to any one of the claims 1 to 13.

35. Use according to claim 20 for a composition which is to be applied at a temperature between 20 and 45°C to said tissues and/or to said biological material, said biological material having functions, particularly amino functions, which are reactive with respect to said polyaldehyde.

36. Use according to claim 35 for bonding a collagen lens.

37. Use according to claim 21 for a composition which:
- is mixed at a neutral, physiological pH with an aqueous solution of a collagenic component chosen from among
• collagen having at least partly lost its helical structure and non-hydrolyzed, largely constituted by α chains,
• native collagen in a concentration below 5%, and an aqueous solution containing at least one natural, biodegradable, macromolecular polyaldehyde,
- the gel obtained being rapidly applied to said tissues and/or said biological material, the latter having functions, particularly amino functions, reactive with respect to said macromolecular polyaldehydes and at a temperature between 20 and 45°C and
- said mixture is allowed to polymerize.

38. Use according to any one of the claims 35 to 37, **characterized in that** use is made of an aqueous polyaldehyde solution at the naturally obtained acid pH.

39. Use according to any one of the claims 35 to 37, **characterized in that** use is made of an aqueous polyaldehyde solution at the previously neutralized pH.

40. Use according to any one of the claims 37 to 39, **characterized in that,** in the case of collagen having at least partly lost its helical structure and non-hydrolyzed and largely constituted by a chains, application takes place at a temperature between 37 and 42°C.

41. Use according to any one of the claims 37 to 39, **characterized in that,** in the case of native collagen, application takes place at ambient temperature.

42. Use according to any one of the claims 35 to 41, **characterized in that** use is made of a composition according to any one of the claims 1 to 13.

43. Use according to any one of the claims to 42, **characterized in that** use is made of a kit according to one of the claims 23 to 34.

44. Use according to any one of the claims 36 to 43, for the prevention of post-operative adhesions.

## Patentansprüche

1. Klebstoffzusammensetzung, die biologisch verträglich, bioresorbierbar und nicht toxisch ist, für eine chirurgische und/oder therapeutische Verwendung, insbesondere für die Verbindung von biologischen Geweben untereinander oder mit einem implantierten Biomaterial, umfassend mindestens ein biologisch abbaubares, makromolekulares polyaldehydisches oxidiertes Polysaccharid oder Mucopolysaccharid natürlicher Herkunft in wässriger Lösung, **dadurch gekennzeichnet, dass** sie mindestens ein biologisch abbaubares, makromolekulares Polyaldehyd natürlicher Herkunft in wässriger Lösung mit saurem pH-Wert, der auf natürliche Weise erhalten worden ist, umfasst.

2. Klebstoffzusammensetzung, die biologisch verträglich, bioresorbierbar und nicht-toxisch ist, für eine chirurgische und/oder therapeutische Verwendung, insbesondere für die Verbindung von biologischem Gewebe untereinander oder mit einem implantierten Biomaterial, **dadurch gekennzeichnet, dass** sie
- mindestens ein biologisch abbaubares, makromolekulares polyaldehydisches oxidiertes Polysaccharid oder Mucopolysaccharid natürlicher Herkunft und
- eine Collagen-Komponente umfasst, die im wässrigen Medium solubilisiert und aus
• Collagen, das nicht hydrolysiert ist, wenigstens teilweise seine Helixstruktur verloren hat und überwiegend aus einer α-Kette besteht, wobei diese Zusammensetzung bei ihrer endgültigen Verwendung nur eine Erwärmung erfordert, welche die Herstellung der Klebstoffzusammensetzung durch Vermischen und anschließend ihr Aufbringen auf das Gewebe bei einer Temperatur von 20 bis 45°C erlaubt, und
• nativem Collagen mit einer Konzentration von unter 5 %, wobei die Zusammensetzung dann die endgültige Verwendung bei Umgebungstemperatur erlaubt,
ausgewählt ist.

3. Klebstoffzusammensetzung nach Anspruch 2, **dadurch gekennzeichnet, dass** sie mindestens ein biologisch abbaubares, makromolekulares Polyaldehyd natürlicher Herkunft in wässriger Lösung mit saurem pH-Wert, der auf natürliche Weise erhalten worden ist, umfasst.

4. Klebstoffzusammensetzung nach einem der vorhergehenden Ansprüche, **dadurch gekennzeichnet, dass** das Polysaccharid oder Mucopolysaccharid aus der Gruppe ausgewählt ist, die Stärke, Dextran, Agarose, Cellulose, Chitin, Chitosan, Alginsäure, Glykosaminoglykane, Hyaluronsäure und Chondroitinsulfat oder ihre Derivate umfasst.

5. Klebstoffzusammensetzung nach einem der vorhergehenden Ansprüche, **dadurch gekennzeichnet, dass** das Polysaccharid aus Stärke und Dextran ausgewählt und vorzugsweise Stärke ist.

6. Klebstoffzusammensetzung nach einem der vorhergehenden Ansprüche, **dadurch gekennzeichnet, dass** das Polyaldehyd aus einem Polysaccharid oder Mucopolysaccharid besteht, das durch Periodsäure oder eines ihrer Salze oxidiert ist.

7. Klebstoffzusammensetzung nach einem der vorhergehenden Ansprüche, **dadurch gekennzeichnet, dass** das Polysaccharid ein Molekulargewicht von etwa 10 000 bis 2 Millionen Dalton besitzt.

8. Klebstoffzusammensetzung nach einem der vorhergehenden Ansprüche, **dadurch gekennzeichnet, dass** sie eine wässrige Lösung von Polyaldehyd(en) mit einer Konzentration von 0,5 bis 5 Gew.-% umfasst.

9. Klebstoffzusammensetzung nach einem der vorhergehenden Ansprüche, **dadurch gekennzeichnet, dass** die wässrige Lösung durch Auflösung eines Polyaldehyds in saurer gefriergetrockneter Form in Wasser oder mit einem physiologischen Puffer erhalten worden ist.

10. Klebstoffzusammensetzung nach einem der Ansprüche 2 bis 9, **dadurch gekennzeichnet, dass** sie eine Lösung von Collagen, das seine Helixstruktur wenigstens teilweise verloren hat und nicht hydrolysiert ist, mit einer Konzentration von 10 bis 20 % und vorzugsweise 15 bis 18 % umfasst.

11. Klebstoffzusammensetzung nach einem der Ansprüche 2 bis 9, **dadurch gekennzeichnet, dass** sie eine wässrige Lösung von nativem Collagen mit einer Konzentration von 0,2 bis 5 % und vorzugsweise 1 bis 3 % umfasst.

12. Klebstoffzusammensetzung nach einem der Ansprüche 2 bis 11, **dadurch gekennzeichnet, dass** zum Vermischen der Polyaldehydanteil, bezogen auf das Collagen, 0,5 bis 10 Gew.-% und vorzugsweise 3 bis 10 Gew.-% beträgt.

13. Klebstoffzusammensetzung nach einem der vorhergehenden Ansprüche, **dadurch gekennzeichnet, dass** sie im Organismus innerhalb von 1 bis 2 Tagen und 30 bis 60 Tagen resorbierbar ist.

14. Verfahren zur Herstellung eines nicht-toxischen, bioresorbierbaren und biologisch verträglichen Klebstoffs für eine chirurgische und/oder therapeutische Verwendung, der vorgesehen ist, auf Gewebe und/oder ein Biomaterial aufgebracht zu werden, **dadurch gekennzeichnet, dass** es eine Stufe umfasst, die im Vermischen einer im wässrigen Medium solubilisierten Collagenkomponente, die aus
- Collagen, das nicht hydrolysiert ist, wenigstens teilweise seine Helixstruktur verloren hat und im Wesentlichen aus α-Ketten besteht, und
- nativem Collagen mit einer Konzentration von unter 5 % ausgewählt ist, vor ihrer Vernetzung mit mindestens einem biologisch abbaubaren, makromolekularen polyaldehydischen oxidierten Polysaccharid oder Mucopolysaccharid natürlicher Herkunft in wässriger Lösung besteht.

15. Verfahren nach Anspruch 14, **dadurch gekennzeichnet, dass** die Lösung der Collagenkomponente und die Lösung des makromolekularen Polyaldehyds wie in einem der Ansprüche 3 bis 13 definiert sind.

16. Verfahren nach Anspruch 14 oder 15, **dadurch gekennzeichnet, dass** das Vermischen bei einer Temperatur von 20 bis 45°C durchgeführt wird.

17. Verfahren nach Anspruch 16, **dadurch gekennzeichnet, dass** das Vermischen bei einer Temperatur von etwa 37 bis 42 °C durchgeführt wird.

18. Verfahren nach einem der Ansprüche 14 bis 17, **dadurch gekennzeichnet, dass** das Vermischen bei einem neutralen, physiologischen pH-Wert durchgeführt wird.

19. Verfahren nach einem der Ansprüche 14 bis 18, **dadurch gekennzeichnet, dass** die Vernetzung innerhalb von weniger als 5 Minuten durchgeführt wird.

20. Verwendung eines biologisch abbaubaren, makromolekularen polyaldehydischen oxidierten Polysaccharids oder Mucopolysaccharids natürlicher Herkunft in wässriger Lösung zur Herstellung einer nicht-toxischen, bioresorbierbaren und biologisch verträglichen Klebstoffzusammensetzung für eine chirurgische und/oder therapeutische Verwendung zur Verbindung von biologischem Gewebe untereinander oder mit einem implantierten Biomaterial, das gegenüber dem Polyaldehyd reaktive Aminfunktionen besitzt.

21. Verwendung nach Anspruch 20 in Verbindung mit einer im wässrigen Medium solubilisierten Collagenkomponente, die aus
- Collagen, das nicht hydrolysiert ist, seine Helixstruktur wenigstens teilweise verloren hat und überwiegend aus α-Ketten besteht, und
- nativem Collagen mit einer Konzentration von unter 5 %
ausgewählt ist.

22. Verwendung nach Anspruch 20 oder 21 zur Herstellung einer Klebstoffzusammensetzung wie in einem der Ansprüche 1 bis 13 definiert.

23. Kit, der eine Zusammensetzung nach Anspruch 1 umfasst.

24. Kit nach Anspruch 23, **dadurch gekennzeichnet, dass** er in Form einer Spritze vorliegt.

25. Kit nach Anspruch 23 oder 24, **dadurch gekennzeichnet, dass** er eine wässrige Lösung von Polyaldehyd(en) mit einer Konzentration von 0,5 bis 5 Gew.-% umfasst.

26. Kit nach Anspruch 23, **dadurch gekennzeichnet, dass** er außerdem
- eine wässrige Lösung einer Collagenkomponente, die aus
• Collagen, das nicht hydrolysiert ist, seine Helixstruktur wenigstens teilweise verloren hat und überwiegend aus α-Ketten besteht, und
• nativem Collagen mit einer Konzentration von unter 5 % ausgewählt ist, und
- Mittel zum sofortigen Vermischen dieser Lösungen umfasst.

27. Kit nach Anspruch 26, **dadurch gekennzeichnet, dass** er in Form von zwei Spritzen vorliegt, die mit Mischmitteln versehen sind, wobei eine der Spritzen die Lösung der Collagenkomponente und die andere die Lösung des makromolekularen Polyaldehyds enthält.

28. Kit nach Anspruch 26 und 27, **dadurch gekennzeichnet, dass** er eine 0,5- bis 5%ige Polyaldehydlösung enthält.

29. Kit nach einem der Ansprüche 23 bis 28, **dadurch gekennzeichnet, dass** die Polyaldehydlösung durch Auflösung eines Polyaldehyds in saurer gefriergetrockneter Form in Wasser oder mit einem physiologischen Puffer erhalten worden ist.

30. Kit nach einem der Ansprüche 26 bis 29, **dadurch gekennzeichnet, dass** er eine 10- bis 20%ige und vorzugsweise 15- bis 18%ige Lösung von Collagen, das nicht hydrolysiert ist, seine Helixstruktur wenigstens teilweise verloren hat.und überwiegend aus α-Ketten besteht, enthält.

31. Kit nach einem der Ansprüche 26 bis 30, **dadurch gekennzeichnet, dass** er einerseits Collagen, das nicht hydrolysiert ist, seine Helixstruktur wenigstens teilweise verloren hat und überwiegend aus α-Ketten besteht, in 18%iger wässriger Lösung und andererseits eine 3%ige wässerige Lösung von oxidierter Stärke mit einem Verhältnis von 1/24 umfasst.

32. Kit nach einem der Ansprüche 26 bis 29, **dadurch gekennzeichnet, dass** er eine wässrige Lösung von nativem Collagen mit einer Konzentration von 0,2 bis 5 % und vorzugsweise 1 bis 3 % umfasst.

33. Kit nach einem der Ansprüche 26 bis 32, **dadurch gekennzeichnet, dass** die Lösung(en) auf einer Temperatur von +1 bis +25 °C und vorzugsweise +2 bis +8 °C gehalten werden.

34. Kit nach einem der Ansprüche 23 bis 33, **dadurch gekennzeichnet, dass** er eine Zusammensetzung nach einem der Ansprüche 1 bis 13 enthält.

35. Verwendung nach Anspruch 20 einer Zusammensetzung, die vorgesehen ist, bei einer Temperatur von 20 bis 45 °C auf Gewebe und/oder Biomaterial aufgebracht zu werden, wobei das Biomaterial Funktionen, insbesondere Aminfunktionen, besitzt, die gegenüber dem Polyaldehyd reaktionsfähig sind.

36. Verwendung nach Anspruch 35 zur Verbindung mit einer Collagenlinse.

37. Verwendung nach Anspruch 21 einer Zusammensetzung derart, dass
- bei neutralem, physiologischem pH-Wert eine wässrige Lösung einer Collagenkomponente, die aus
• Collagen, das nicht hydrolysiert ist, seine Helixstruktur wenigstens teilweise verloren hat und überwiegend aus α-Ketten besteht, und
• nativem Collagen mit einer Konzentration von unter 5 % ausgewählt ist, mit
einer wässrigen Lösung, die mindestens ein biologisch abbaubares, makromolekulares Polyaldehyd natürlicher Herkunft enthält, vermischt wird, wobei
- das erhaltene Gel auf das Gewebe und/oder Biomaterial, das Funktionen, insbesondere Aminfunktionen, die mit den makromolekularen Polyaldehyden reaktionsfähig sind, besitzt, bei einer Temperatur von 20 bis 45 °C schnell aufgebracht und
- das Gemisch polymerisieren gelassen wird.

38. Verwendung nach einem der Ansprüche 35 bis 37, **dadurch gekennzeichnet, dass** eine wässrige Polyaldehydlösung mit einem auf natürliche Weise erhaltenen sauren pH-Wert verwendet wird.

39. Verwendung nach einem der Ansprüche 35 bis 37, **dadurch gekennzeichnet, dass** eine wässrige Polyaldehydlösung mit zuvor neutralisiertem pH-Wert verwendet wird.

40. Verwendung nach einem der Ansprüche 37 bis 39, **dadurch gekennzeichnet, dass** das Collagen, das nicht hydrolysiert ist, seine Helixstruktur wenigstens teilweise verloren hat und überwiegend aus α-Ketten besteht, bei einer Temperatur von 37 bis 42 °C aufgebracht wird.

41. Verwendung nach einem der Ansprüche 37 bis 39, **dadurch gekennzeichnet, dass** das native Collagen bei Umgebungstemperatur aufgebracht wird.

42. Verwendung nach einem der Ansprüche 35 bis 41, **dadurch gekennzeichnet, dass** eine Zusammensetzung nach einem der Ansprüche 1 bis 13 verwendet wird.

43. Verwendung nach einem der Ansprüche 36 bis 42, **dadurch gekennzeichnet, dass** ein Kit nach einem der Ansprüche 23 bis 34 verwendet wird.

44. Verwendung nach einem der Ansprüche 36 bis 43 zur Verhinderung postoperativer Anhaftungen.
